# EUROPÄISCHE PATENTSCHRIFT

(11) **EP 2 854 765 B1**
(45) Veröffentlichungstag und Bekanntmachung des Hinweises auf die Patenterteilung: **27.03.2019**
(21) Anmeldenummer: 13737527.5
(22) Anmeldetag: 29.05.2013
(51) Int. Cl.: A61K 9/08, A61K 9/00, A61K 47/18, A61K 47/20, A61K 31/519

(54) **PHARMAZEUTISCHE PEMETREXED-LÖSUNG**
PHARMACEUTICAL PEMETREXED SOLUTION
SOLUTION PHARMACEUTIQUE DE PEMETREXED

(30) Priorität: 31.05.2012 DE 102012010774
(43) Veröffentlichungstag der Anmeldung: 08.04.2015
(73) Patentinhaber: STADA ARZNEIMITTEL AG, 61118 Bad Vilbel (DE)
(72) Erfinder: SCHRIDDE, Edgar, 30855 Langenhagen (DE)
(74) Vertreter: Kernebeck, Thomas
(86) Internationale Anmeldenummer: PCT/DE2013/000292
(87) Internationale Veröffentlichungsnummer: WO 2013/178214

(56) Entgegenhaltungen:
- EP-B1- 1 265 612
- KR-B1- 101 260 636

## Beschreibung

Die vorliegende Erfindung betrifft eine flüssige pharmazeutische Lösung gemäß Anspruch 1, umfassend
- ein Lösungsmittel;
- Pemetrexed oder ein pharmazeutisch annehmbares Salz davon; sowie
- ein Antioxidationsmittel.

Pemetrexed ist der internationale Freiname (INN (International Nonproprietary Name)) für N-[4-[2-(2-Amino-4,7-dihydro-4-oxo-1H-pyrrolo[2,3-d]pyrimidin-5-yl)ethyl]-benzoyl]-L-glutaminsäure. Pemetrexed weist die folgende Strukturformel auf:

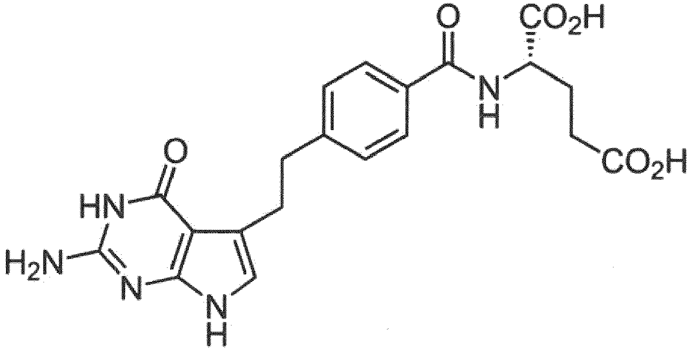

Pemetrexed ist ein Arzneistoff aus der Gruppe der Folsäureantagonisten, der zur Behandlung von zwei Lungenkrebsarten angewendet wird:
a) malignes Pleuramesotheliom (einer Krebsart des Lungenfells, die in der Regel durch eine Asbestexposition ausgelöst wird), wo es zusammen mit Cisplatin bei Patienten angewendet wird, die zuvor keine Chemotherapie erhalten haben und bei denen der Krebs nicht durch eine Operation entfernt werden kann.
b) fortgeschrittener "nicht-kleinzelliger" Lungenkrebs vom "nicht-squamösen" Typ, wo es entweder zusammen mit Cisplatin bei zuvor unbehandelten Patienten oder allein bei Patienten angewendet wird, die zuvor Arzneimittel gegen Krebs erhalten haben. Es kann auch als Erhaltungstherapie bei Patienten angewendet werden, die eine Chemotherapie auf Platinbasis erhalten haben.

Die Wirkung von Pemetrexed beruht auf der Blockierung sowohl der Thymidilat-Synthase als auch der Dihydrofolat-Reduktase und der Glycinamidribonucleotidformyl-Transferase, wodurch die folat-abhängige Biosynthese von Thymidin und Purinnucleotiden gehemmt wird (vgl. Mutschler Arzneimittelwirkungen, Lehrbuch der Pharmakologie und Toxikologie, 9. Auflage, Wissenschaftliche Verlagsgesellschaft mbH Stuttgart, 2008, S. 919-920; ISBN 978-3-8047-1952-1).

Pemetrexed wird unter der Markenbezeichnung Alimta® in der Darreichungsform eines Lyophilisates in den Wirkstärken 100 mg und 500 mg vertrieben. Für die Verabreichung wird das sterile Lyophilisat zunächst mit einer 0,9 %igen Kochsalzlösung rekonstituiert, um eine Lösung mit 25 mg/ml Pemetrexed zu erhalten, die mit der empfohlenen Dosis von 500 mg/qm Körperoberfläche, und nach weiterer Verdünnung mit 0,9 %iger Kochsalzlösung auf 100 ml verdünnt, innerhalb 10 Minuten intravenös verabreicht wird. Die chemische und physikalische Stabilität der durch Rekonstitution des Lyophilisates erhaltenen Pemetrexed-Lösung und der daraus hergestellten Infusionslösung wird angegeben mit nur 24 Stunden.

Im Hinblick auf eine sicherere Handhabung des Zytostatikums Pemetrexed wäre es wünschenswert, wenn man Pemetrexed in Form einer Lösung darreichen könnte. EP 1 265 612 B1 schlägt hierzu eine pharmazeutische Lösung, umfassend ein flüssiges Lösungsmittel, Pemetrexed oder ein pharmazeutisch annehmbares Salz davon sowie ein Antioxidationsmittel, ausgewählt aus der Gruppe bestehend aus Monothioglycerin, L-Cystein und Thioglycolsäure vor. Zwar ist der Wirkstoff in diesen Lösungen gegenüber oxidativem Abbau stabil, Monothioglycerin sowie Thioglycolsäure sind jedoch toxikologisch sehr bedenklich und im Europäischen Arzneibuch entsprechend nicht monographiert und bezüglich Cystein wurde festgestellt, dass die Lösungen leider nur für verhältnismäßig kurze Zeit gelagert werden können. Und zwar werden die Lösungen aufgrund von Partikelbildung relativ schnell trüb und es kann das Ausfallen eines Niederschlags beobachtet werden. Die Lösungen sind dann für eine intravenöse Injektion ungeeignet.

Aufgabe der vorliegenden Erfindung ist es daher, eine pharmazeutische Lösung der gattungsgemäßen Art bereitzustellen, die toxikologisch unbedenklich ist, eine verhältnismäßig hohe Stabilität aufweist und die für verhältnismäßig lange Zeit gelagert werden kann, ohne dass sie eintrübt oder aus ihr gar ein Niederschlag ausfällt.

Diese Aufgabe wird ausgehend von einer flüssigen pharmazeutischen Lösung der eingangs genannten Art dadurch gelöst, dass das Antioxidationsmittel ausgewählt ist aus der Gruppe bestehend aus Acetylcystein und 2-Mercaptoethansulfonat-Natrium.

Überraschender Weise wurde festgestellt, dass eine flüssige pharmazeutische Lösung, umfassend ein Lösungsmittel, in welchem Pemetrexed oder ein pharmazeutisch annehmbares Salz davon sowie ein Antioxidationsmittel, ausgewählt aus der Gruppe bestehend aus Acetylcystein und 2-Mercaptoethansulfonat-Natrium, gelöst enthalten ist, toxikologisch unbedenklich ist, eine verhältnismäßig hohe Stabilität aufweist und für verhältnismäßig lange Zeit gelagert werden kann, ohne dass sie eintrübt oder aus ihr gar ein Niederschlag ausfällt.

Unter dem Begriff "Stabilität" wird in diesem Zusammenhang die von der Arbeitsgemeinschaft für pharmazeutische Verfahrenstechnik (APV) erarbeitete Definition verstanden, nach welcher "Stabilität" die spezifikationsgerechte Qualität des Arzneimittels bis zum Ende der vom Hersteller festgelegten Laufzeit bedeutet. Die Qualität des Arzneimittels wird dabei durch den Wirkstoffgehalt und die Reinheit, die sensorisch wahrnehmbaren, physikalisch-chemischen und die mikrobiologischen Eigenschaften bestimmt, wobei der Wirkstoffgehalt bis zum Ende der Laufzeit 90 % des deklarierten Wertes nicht unterschreiten soll.

Ferner wurde festgestellt, dass sich die erfindungsgemäße Lösung durch eine verhältnismäßig hohe Sterilisationsstabilität auszeichnet. Dadurch ist gewährleistet, dass die erfindungsgemäße Lösung gemäß den gesetzlichen Bestimmungen steril angeboten werden kann. Eine typische Sterilisation kann beispielsweise bei einer Temperatur von 121 °C über einen Zeitraum von 15 min in einem Autoklaven durchgeführt werden.

Darüber hinaus zeichnet sich die erfindungsgemäße Lösung durch eine verhältnismäßig gute Patientenverträglichkeit aus.

Die erfindungsgemäße Lösung ist zur parenteralen Verabreichung geeignet, beispielsweise zur intravenösen Verabreichung. Zur parenteralen Verabreichung kann die erfindungsgemäße Lösung sowohl unmittelbar als auch nach weiterer Zubereitung verabreicht werden. Zur unmittelbaren Verabreichung kann die erfindungsgemäße Lösung beispielsweise intravenös injiziert werden. Die weitere Zubereitung der erfindungsgemäßen Lösung kann beispielsweise vorsehen, dass die Lösung vor einer parenteralen Verabreichung mittels einer Trägerlösung, wie z.B. einer Kochsalzlösung, auf eine gewünschte Wirkstoffkonzentration, auf einen gewünschten pH-Wert, usw. eingestellt wird.

Gemäß einer bevorzugten Ausführungsform der erfindungsgemäßen Lösung ist es vorgesehen, dass das Lösungsmittel ausgewählt ist aus der Gruppe bestehend aus Wasser, Polyethylenglycol und Ethanol sowie aus Mischungen von zwei oder mehr der genannten Lösungsmittel. Es konnte festgestellt werden, dass die genannten Lösungsmittel bzw. Lösungsmittelgemische gute Lösungseigenschaften für Wirkstoff und Antioxidans aufweisen, die Stabilität des Wirkstoffs nicht nachteilig beeinflussen und eine Partikelbildung in der Lösung nicht fördern.

Erfindungsgemäß besonders bevorzugt ist es, wenn das Lösungsmittel Wasser ist.

Entsprechend einer weiteren bevorzugten Ausführungsform der erfindungsgemäßen Lösung ist es vorgesehen, dass das Pemetrexed in der Lösung in Form seines Dinatriumsalzes gelöst enthalten ist. Das Pemetrexed-Dinatriumsalz weist verhältnismäßig gute Löslichkeitseigenschaften auf. So können mittels des Pemetrexed-Dinatriumsalzes beispielsweise erfindungsgemäße Lösungen mit einer relativ hohen Pemetrexedkonzentration bereitgestellt werden, die eine hohe Stabilität des Wirkstoffs zeigen und nicht zur Eintrübung oder zur Ausbildung von Niederschlag neigen. Derartige Konzentrate besitzen eine hohe Akzeptanz beim Patienten und können vom medizinischen Personal einfach und sicher gehandhabt werden. Zur Verabreichung derartiger Konzentrate werden diese einfach mittels einer Trägerlösung auf die gewünschte Wirkstoffkonzentration verdünnt.

Nach einer weiteren bevorzugten Ausführungsform der erfindungsgemäßen Lösung ist es vorgesehen, dass der Gehalt der Lösung an Pemetrexed bzw. an pharmazeutisch annehmbaren Salz davon (bezogen auf das freie Pemetrexed) 0,1 mg/ml bis 100 mg/ml beträgt, mehr bevorzugt 5 mg/ml bis 80 mg/ml, noch mehr bevorzugt 10 mg/ml bis 50 mg/ml und weiter bevorzugt 20 mg/ml bis 40 mg/ml. In den genannten Konzentrationsintervallen zeigt die erfindungsgemäße Lösung eine nur relativ geringe Tendenz zur Partikelbildung und eine damit verbundene Eintrübung der Lösung oder Ausbildung von Niederschlag.

Gemäß einer weiteren bevorzugten Ausführungsform der erfindungsgemäßen Lösung ist es vorgesehen, dass der Gehalt der Lösung an Pemetrexed bzw. an pharmazeutisch annehmbaren Salz davon (bezogen auf das freie Pemetrexed) 10 mg/ml, 20 mg/ml, 25 mg/ml, 40 mg/ml oder 50 mg/ml beträgt.

Entsprechend einer weiteren bevorzugten Ausführungsform der erfindungsgemäßen Lösung ist es vorgesehen, dass das Antioxidationsmittel Acetylcystein ist. Es konnte festgestellt werden, dass Acetylcystein (oder anders N-Acetyl-L-cystein) in der erfindungsgemäßen Lösung eine verhältnismäßig hohe Stabilität des Pemetrexeds bewirkt und dass eine entsprechende Lösung verhältnismäßig lange Zeit gelagert werden kann, ohne dass sie eintrübt oder aus ihr gar ein Niederschlag ausfällt.

Nach einer weiteren bevorzugten Ausführungsform der erfindungsgemäßen Lösung ist es vorgesehen, dass, wenn Acetylcystein als Antioxidationsmittel enthalten ist, die Lösung einen pH-Wert in einem Bereich von 7,5 bis 11,5 aufweist, mehr bevorzugt einen pH-Wert in einem Bereich von 8,0 bis 10,5 und noch mehr bevorzugt einen pH-Wert in einem Bereich von 8,5 bis 10,5. Es wurde festgestellt, dass der Wirkstoff in der erfindungsgemäßen Lösung in den genannten pH-Wertintervallen durch eine verhältnismäßig hohe Stabilität insbesondere gegenüber oxidativem Abbau gekennzeichnet ist.

Nach einer weiteren bevorzugten Ausführungsform der erfindungsgemäßen Lösung ist es vorgesehen, dass das Antioxidationsmittel 2-Mercaptoethansulfonat-Natrium ist. Es konnte festgestellt werden, dass 2-Mercaptoethansulfonat-Natrium in der erfindungsgemäßen Lösung eine verhältnismäßig hohe Stabilität des Pemetrexeds bewirkt und dass eine entsprechende Lösung verhältnismäßig lange Zeit gelagert werden kann, ohne dass sie eintrübt oder aus ihr gar ein Niederschlag ausfällt.

Gemäß einer weiteren bevorzugten Ausführungsform der erfindungsgemäßen Lösung ist es vorgesehen, dass, wenn 2-Mercaptoethansulfonat-Natrium als Antioxidationsmittel enthalten ist, die Lösung einen pH-Wert in einem Bereich von 7,5 bis 11,5 aufweist, mehr bevorzugt einen pH-Wert in einem Bereich von 8,0 bis 10,5, noch mehr bevorzugt einen pH-Wert in einem Bereich von 8,0 bis 10,0 und weiter bevorzugt einen pH-Wert in einem Bereich von 8,3 bis 9,3. Es wurde festgestellt, dass der Wirkstoff in der erfindungsgemäßen Lösung in den genannten pH-Wertintervallen durch eine verhältnismäßig hohe Stabilität insbesondere gegenüber oxidativem Abbau gekennzeichnet ist.

Entsprechend einer weiteren bevorzugten Ausführungsform der erfindungsgemäßen Lösung ist es vorgesehen, dass der Gehalt der Lösung an Antioxidationsmittel 0,1 mg/ml bis 100 mg/ml beträgt, mehr bevorzugt 0,5 mg/ml bis 20 mg/ml, noch mehr bevorzugt 1,0 mg/ml bis 5 mg/ml und weiter bevorzugt 1 mg/ml bis 3 mg/ml. Es konnte festgestellt werden, dass der Wirkstoff in der erfindungsgemäßen Lösung in den genannten Konzentrationsintervallen durch eine verhältnismäßig hohe Stabilität insbesondere gegenüber oxidativem Abbau gekennzeichnet ist.

Nach einer weiteren bevorzugten Ausführungsform der erfindungsgemäßen Lösung ist es vorgesehen, dass die Lösung ferner einen oder mehrere pharmazeutische Hilfsstoffe umfasst, ausgewählt aus der Gruppe bestehend aus Salzen, Kohlehydraten zur Tonisierung, Chelatbildnern zur Komplexierung von Schwermetallen, Säuren zur pH-Wert Einstellung, Basen zur pH-Wert Einstellung, Puffersubstanzen, Konservierungsmitteln zur mikrobiellen Konservierung der Lösung. Die genannten Substanzen sind dem zuständigen Fachmann aus dem Stand der Technik hinlänglich bekannt.

Die vorliegende Erfindung betrifft ferner eine pharmazeutische Zusammensetzung zur Herstellung der erfindungsgemäßen Lösung, umfassend Pemetrexed oder ein pharmazeutisch annehmbares Salz davon sowie ein Antioxidationsmittel ausgewählt aus der Gruppe bestehend aus Acetylcystein und 2-Mercaptoethansulfonat-Natrium. Mittels der erfindungsgemäßen Zusammensetzung kann die erfindungsgemäße Lösung leicht durch Aufnahme der Zusammensetzung in einer Trägerflüssigkeit hergestellt werden.

Gemäß einer bevorzugten Ausführungsform der erfindungsgemäßen Zusammensetzung ist es vorgesehen, dass die Zusammensetzung in fester Form vorliegt. Es konnte festgestellt werden, dass der Wirkstoff in der erfindungsgemäßen Zusammensetzung in fester Form durch eine sehr hohe Stabilität gekennzeichnet ist.

Entsprechend einer weiteren bevorzugten Ausführungsform der erfindungsgemäßen Zusammensetzung ist es vorgesehen, dass die Zusammensetzung ein Lyophilisat ist. Dadurch wird die Möglichkeit zum leichten und vollständigen Rekonstituieren der erfindungsgemäßen Zusammensetzung zur erfindungsgemäßen Lösung gegeben.

Nach einer weiteren bevorzugten Ausführungsform der erfindungsgemäßen Zusammensetzung ist es vorgesehen, dass die Zusammensetzung ferner einen Gerüstbildner umfasst. Dadurch ist die Möglichkeit gegeben, die erfindungsgemäße Zusammensetzung über einen verhältnismäßig langen Zeitraum leicht und vollständig zur erfindungsgemäßen Lösung rekonstituieren zu können. Ein erfindungsgemäß bevorzugter Gerüstbildner ist beispielsweise Mannitol.

Die vorliegende Erfindung betrifft ferner einen luftdicht verschlossenen Behälter, enthaltend eine erfindungsgemäße pharmazeutische Lösung oder eine erfindungsgemäße pharmazeutische Zusammensetzung. Die in dem erfindungsgemäßen Behälter enthaltene Lösung bzw. Zusammensetzung ist durch eine sehr hohe Stabilität hinsichtlich des Wirkstoffs gekennzeichnet.

Gemäß einer bevorzugten Ausführungsform des erfindungsgemäßen luftdicht verschlossenen Behälters ist es vorgesehen, dass der Behälter aus einem Material gebildet ist, das für Sauerstoff nicht permeabel ist.

Entsprechend einer weiteren bevorzugten Ausführungsform des erfindungsgemäßen luftdicht verschlossenen Behälters ist es vorgesehen, dass der Behälter aus Glas gebildet ist.

Entsprechend einer weiteren bevorzugten Ausführungsform des erfindungsgemäßen luftdicht verschlossenen Behälters ist es vorgesehen, dass der luftdicht verschlossene Behälter ein Vial ist. Vials sind im Stand der Technik bekannt. Dabei handelt es sich um sogenannte Injektionsflaschen, die häufig als Durchstechflaschen mit Kunststoffstopfen und Alu-Bördelkappe ausgebildet sind, wobei der Kunststoffstopfen in der Mitte seine geringste Dicke zur leichteren Durchstechung aufweist.

Eine typische, durch die vorliegende Erfindung bereitgestellte flüssige pharmazeutische Lösung ist eine Lösung, umfassend Wasser als Lösungsmittel, 10 mg/ml bis 50 mg/ml Pemetrexed-Dinatriumsalz (bezogen auf das freie Pemetrexed) und 0,5 mg/ml bis 20 mg/ml und bevorzugt 1,0 mg/ml bis 5 mg/ml Acetylcystein als Antioxidationsmittel, wobei die Lösung einen pH-Wert von 8,0 bis 10,5 und bevorzugt einen pH-Wert von 8,5 bis 10,5 aufweist.

Eine weitere typische, durch die vorliegende Erfindung bereitgestellte flüssige pharmazeutische Lösung ist eine Lösung, umfassend Wasser als Lösungsmittel, 10 mg/ml bis 50 mg/ml Pemetrexed-Dinatriumsalz (bezogen auf das freie Pemetrexed) und 0,5 mg/ml bis 20 mg/ml und bevorzugt 1,0 mg/ml bis 5 mg/ml 2-Mercaptoethansulfonat-Natrium als Antioxidationsmittel, wobei die Lösung einen pH-Wert von 8,0 bis 10,0 und bevorzugt einen pH-Wert von 8,3 bis 9,3 aufweist.

Die nachstehenden Vergleichs- und Ausführungsbeispiele dienen der Erläuterung der Erfindung.

### Vergleichsbeispiel:

Unter aseptischen Bedingungen wurden zu 350 ml Wasser für Injektionszwecke als Lösungsmittel 5,0 g L-Cystein (Ph. Eur.) als Antioxidationsmittel gegeben und darin klar gelöst unter Erhalt einer ersten Lösung. Zu der so erhaltenen ersten Lösung wurden dann 20,0 g Mannitol (Ph. Eur.) gegeben und darin klar gelöst unter Erhalt einer zweiten Lösung. Der pH-Wert der zweiten Lösung wurde auf einen Wert von 8,5 mittels 1 molarer Natronlauge und 1 molarer Salzsäure eingestellt.

Zu der zweiten Lösung mit einem pH-Wert von 8,5 wurden dann 22,06 g Pemetrexed-Dinatriumsalz (berechnet als wasserfreie Substanz; entsprechend 20,0 g Pemetrexed) gegeben und darin klar gelöst unter Erhalt einer dritten Lösung. Der pH-Wert der dritten Lösung wurde auf einen Wert von 8,5 mittels 1 molarer Natronlauge und 1 molarer Salzsäure eingestellt.

Die resultierende dritte Lösung mit einem pH-Wert von 8,5 wurde dann mit Wasser für Injektionszwecke als Lösungsmittel auf das Endvolumen von 500 ml aufgefüllt unter Erhalt einer vierten Lösung und der pH-Wert dieser Lösung falls erforderlich wieder auf einen Wert von 8,5 mittels 1 molarer Natronlauge und 1 molarer Salzsäure eingestellt.

2,75 ml der so erhaltenen vierten Lösung wurden nach Sterilfiltration in eine Injektionsflasche als Vial unter aseptischen Bedingungen abgefüllt und mit einem Stopfen und Bördelkappe luftdicht verschlossen.

Die nachfolgende Tabelle 1 beschreibt Parameter der pharmazeutischen Lösung des Vergleichsbeispiels in der abgedichteten Injektionsflasche:

**Tabelle 1:**

| | |
|---|---|
| Pemetrexed | 40,00 mg/ml |
| Mannitol | 40,00 mg/ml |
| L-Cystein | 10,00 mg/ml |
| Natriumhydroxid | q.s. |
| Hydrogenchlorid | q.s. |
| Wasser für Injektionszwecke | q.s. ad 1 ml |
| Injektionsflasche | Typ: 6R-DIN |
| Nominalvolumen | 6,00 ml |
| Randvollvolumen | 10,00 ml |
| Abfüllvolumen | 2,75 ml |
| Kopfraumvolumen | 7,25 ml |
| Sauerstoffgehalt in der Injektionsflasche | 21 Vol.-% |
| Gesamtmenge an Sauerstoff in der Injektionsflasche | 67,93 µmol |
| Gesamtmenge an Pemetrexed in der Injektionsflasche | 110,00 mg |
| Gesamtmenge an L-Cystein in der Injektionsflasche | 27,50 mg |

Die befüllte Injektionsflasche wurde bei einer Temperatur von 4 °C über einen Zeitraum von zwei Wochen und danach bei einer Temperatur von 40 °C über einen Zeitraum von 12 Wochen gelagert. Nach der Lagerung wurde der Inhalt der Injektionsflasche optisch beurteilt. Es konnte eine signifikante Niederschlagsbildung festgestellt werden.

### Ausführungsbeispiel 1:

Unter aseptischen Bedingungen wurden zu 350 ml Wasser für Injektionszwecke als Lösungsmittel 5,0 g Acetylcystein (Ph. Eur.) als Antioxidationsmittel gegeben und darin klar gelöst unter Erhalt einer ersten Lösung. Zu der so erhaltenen ersten Lösung wurden dann 20,0 g Mannitol (Ph. Eur.) gegeben und darin klar gelöst unter Erhalt einer zweiten Lösung. Der pH-Wert der zweiten Lösung wurde auf einen Wert von 8,5 mittels 1 molarer Natronlauge und 1 molarer Salzsäure eingestellt.

Zu der zweiten Lösung mit einem pH-Wert von 8,5 wurden dann 22,06 g Pemetrexed-Dinatriumsalz (berechnet als wasserfreie Substanz; entsprechend 20,0 g Pemetrexed) gegeben und darin klar gelöst unter Erhalt einer erfindungsgemäßen Lösung. Der pH-Wert der erfindungsgemäßen Lösung wurde auf einen Wert von 8,5 mittels 1 molarer Natronlauge und 1 molarer Salzsäure eingestellt.

Die resultierende erfindungsgemäßen Lösung mit einem pH-Wert von 8,5 wurde dann mit Wasser für Injektionszwecke als Lösungsmittel auf das Endvolumen von 500 ml aufgefüllt unter Erhalt einer weiteren erfindungsgemäßen Lösung und der pH-Wert dieser Lösung falls erforderlich wieder auf einen Wert von 8,5 mittels 1 molarer Natronlauge und 1 molarer Salzsäure eingestellt.

2,75 ml der so erhaltenen weiteren erfindungsgemäßen Lösung wurden nach Sterilfiltration in eine Injektionsflasche als Vial unter aseptischen Bedingungen abgefüllt und mit einem Stopfen und Bördelkappe luftdicht verschlossen unter Erhalt eines erfindungsgemäßen, luftdicht verschlossenen Behälters.

Die nachfolgende Tabelle 2 beschreibt Parameter der erfindungsgemäßen pharmazeutischen Lösung des Ausführungsbeispiels 1 in dem erfindungsgemäßen, luftdicht verschlossenen Behälter:

**Tabelle 2:**

| | |
|---|---|
| Pemetrexed | 40,00 mg/ml |
| Mannitol | 40,00 mg/ml |
| Acetylcystein | 10,00 mg/ml |
| Natriumhydroxid | q.s. |
| Hydrogenchlorid | q.s. |
| Wasser für Injektionszwecke | q.s. ad 1 ml |
| Injektionsflasche | Typ: 6R-DIN |
| Nominalvolumen | 6,00 ml |
| Randvollvolumen | 10,00 ml |
| Abfüllvolumen | 2,75 ml |
| Kopfraumvolumen | 7,25 ml |
| Sauerstoffgehalt in der Injektions-flasche | 21 Vol.-% |
| Gesamtmenge an Sauerstoff in der Injektionsflasche | 67,93 µmol |
| Gesamtmenge an Pemetrexed in der Injektionsflasche | 110,00 mg |
| Gesamtmenge an Acetylcystein in der Injektionsflasche | 27,50 mg |

Die befüllte Injektionsflasche wurde bei einer Temperatur von 4 °C über einen Zeitraum von zwei Wochen und danach bei einer Temperatur von 40 °C über einen Zeitraum von 12 Wochen gelagert. Nach der Lagerung wurde der Inhalt der Injektionsflasche optisch beurteilt. Es konnte keine Eintrübung der erfindungsgemäßen Lösung oder ein Niederschlag in derselben festgestellt werden.

### Ausführungsbeispiel 2:

Unter aseptischen Bedingungen wurden zu 350 ml Wasser für Injektionszwecke als Lösungsmittel 5,0 g 2-Mercaptoethansulfonat-Natrium (Ph. Eur.) als Antioxidationsmittel gegeben und darin klar gelöst unter Erhalt einer ersten Lösung. Zu der so erhaltenen ersten Lösung wurden dann 20,0 g Mannitol (Ph. Eur.) gegeben und darin klar gelöst unter Erhalt einer zweiten Lösung. Der pH-Wert der zweiten Lösung wurde auf einen Wert von 8,5 mittels 1 molarer Natronlauge und 1 molarer Salzsäure eingestellt.

Zu der zweiten Lösung mit einem pH-Wert von 8,5 wurden dann 22,06 g Pemetrexed-Dinatriumsalz (berechnet als wasserfreie Substanz; entsprechend 20,0 g Pemetrexed) gegeben und darin klar gelöst unter Erhalt einer erfindungsgemäßen Lösung. Der pH-Wert der erfindungsgemäßen Lösung wurde auf einen Wert von 8,5 mittels 1 molarer Natronlauge und 1 molarer Salzsäure eingestellt.

Die resultierende erfindungsgemäßen Lösung mit einem pH-Wert von 8,5 wurde dann mit Wasser für Injektionszwecke als Lösungsmittel auf das Endvolumen von 500 ml aufgefüllt unter Erhalt einer weiteren erfindungsgemäßen Lösung und der pH-Wert dieser Lösung falls erforderlich wieder auf einen Wert von 8,5 mittels 1 molarer Natronlauge und 1 molarer Salzsäure eingestellt.

2,75 ml der so erhaltenen weiteren erfindungsgemäßen Lösung wurden nach Sterilfiltration in eine Injektionsflasche als Vial unter aseptischen Bedingungen abgefüllt und mit einem Stopfen und Bördelkappe luftdicht verschlossen unter Erhalt eines erfindungsgemäßen, luftdicht verschlossenen Behälters.

Die nachfolgende Tabelle 3 beschreibt Parameter der erfindungsgemäßen pharmazeutischen Lösung des Ausführungsbeispiels 2 in dem erfindungsgemäßen, luftdicht verschlossenen Behälter:

**Tabelle 3:**

| | |
|---|---|
| Pemetrexed | 40,00 mg/ml |
| Mannitol | 40,00 mg/ml |
| 2-Mercaptoethansulfonat-Natrium | 10,00 mg/ml |
| Natriumhydroxid | q.s. |
| Hydrogenchlorid | q.s. |
| Wasser für Injektionszwecke | q.s. ad 1 ml |
| Injektionsflasche | Typ: 6R-DIN |
| Nominalvolumen | 6,00 ml |
| Randvollvolumen | 10,00 ml |
| Abfüllvolumen | 2,75 ml |
| Kopfraumvolumen | 7,25 ml |
| Sauerstoffgehalt in der Injektions-flasche | 21 Vol.-% |
| Gesamtmenge an Sauerstoff in der Injektionsflasche | 67,93 µmol |
| Gesamtmenge an Pemetrexed in der Injektionsflasche | 110,00 mg |
| Gesamtmenge an 2-Mercaptoethansulfonat-Natrium in der Injektionsflasche | 27,50 mg |

Die befüllte Injektionsflasche wurde bei einer Temperatur von 4 °C über einen Zeitraum von zwei Wochen und danach bei einer Temperatur von 40 °C über einen Zeitraum von 12 Wochen gelagert. Nach der Lagerung wurde der Inhalt der Injektionsflasche optisch beurteilt. Es konnte keine Eintrübung der erfindungsgemäßen Lösung oder ein Niederschlag in derselben festgestellt werden.

### Ausführungsbeispiele 3 und 4:

Pemetrexed-Konzentrate (25 mg/ml) für 100 mg- und 500 mg-Infusionslösungen

### Ausführungsbeispiel 3: für die aseptische Herstellung; 1 ml enthält:

| | |
|---|---|
| Pemetrexed-Dinatrium | 27,57 mg (bezogen auf die wasserfreie Substanz) |
| Mannitol | 25,00 mg |
| Acetylcystein | 2,50 mg |
| Natriumhydroxid | 0,682 mg-0,712 mg |
| Hydrogenchlorid | 0 mg-0,027 mg |
| Wasser für Injektionszwecke | 962,22 mg |
| Stickstoff | |
| Lösungsdichte | 1,018 g/ml |

### Ausführungsbeispiel 4: für den Standardprozess (terminale Sterilisation im Endbehältnis); 1 ml enthält:

| | |
|---|---|
| Pemetrexed-Dinatrium | 27,57 mg (bezogen auf die wasserfreie Substanz) |
| Mannitol | 25,00 mg |
| Acetylcystein | 3,00 mg |
| Natriumhydroxid | 0,818 mg-0,854 mg |
| Hydrogenchlorid | 0 mg-0,032 mg |
| Wasser für Injektionszwecke | 963,58 mg |
| Stickstoff | |
| Lösungsdichte | 1,020 g/ml |

Für die Herstellung der Zusammensetzung gemäß Ausführungsbeispiel 3 und 4 im 30 Liter Ansatz wurden 90 % der benötigten Menge Wasser für Injektionszwecke (20 °C bis 25 °C) im Ansatzkessel vorgelegt und anschließend mindestens 10 Minuten mit Stickstoff begast, bis der Sauerstoffgehalt bei < 0,5 mg/l lag.

Die Gesamtmenge an Acetylcystein wurde unter Rühren in die Vorlage eingebracht und bis zur vollständigen Auflösung gerührt. Danach wurde die Gesamtmenge an Mannitol unter Rühren in die Vorlage eingebracht und bis zur vollständigen Auflösung gerührt. Der pH-Wert wurde mit 10 %iger (w/w) Natriumhydroxidlösung und ggfs. mit 10 %iger (w/w) Salzsäure auf einen pH-Wert von 9,0 +/- 0,1 eingestellt.

Danach wurde die Gesamtmenge an Pemetrexed-Dinatrium unter Rühren in die so erhaltene Lösung eingebracht und bis zur vollständigen Auflösung gerührt. Der pH-Wert wurde dann mit 10 %iger (w/w) Natriumhydroxid-lösung und ggfs. mit 10 %iger (w/w) Salzsäure auf einen pH-Wert von 9,0 +/- 0,1 eingestellt.

Der so erhaltene Ansatz wurde mit Wasser für Injektionszwecke auf das gewünschte Endgewicht/-volumen aufgefüllt und bis zur vollständigen Lösung gerührt. Danach wurde der pH-Wert mit 10 %iger (w/w) Natriumhydroxidlösung und ggfs. mit 10 %iger (w/w) Salzsäure auf pH 9,0 +/- 0,1 eingestellt.

Der so erhaltene Ansatz wurde dann mindestens 10 Minuten mit Stickstoff begast, bis der Sauerstoffgehalt bei < 0,5 mg/l lag.

Die so erhaltene Ansatzlösung wurde im Ansatzkessel mit Stickstoff überlagert und dicht verschlossen. Nach Entnahme der Inprozesskontrollmuster erfolgte die Sterilfiltration der Lösung (20 °C bis 25 °C) über sterilisierte Filtermembrancapsulen mit 0,2 µm Porenweite unter aseptischen Bedingungen in einem sterilen, mit sterilem Stickstoff vorgespülten Lagertank. Die Abfüllung der Lösung (20 °C bis 25 °C) erfolgte unter aseptischen Bedingungen zu jeweils 4,35 ml in hitzesterilisierte 3 ml Sonderform Vial (Fiolaxglas; 100 mg) bzw. zu jeweils 20,50 ml in hitzesterilisierte 20R DIN-Vial (Fiolaxglas; 500 mg).

Während der Abfüllung erfolgte eine Vor-, Füll- und Nachbegasung der Vial mit sterilem Stickstoff. Nach Aufsetzen der Hohlstopfen und Verbördelung der Vial erfolgte nach Außenwaschung, Trocknung und Dichtigkeitsprüfung (Überkopflagerung; 12 Stunden) deren Einlagerung bei 2 °C bis 8 °C (Ausführungsbeispiel 3) bzw. eine terminale Sterilisation im Endbehältnis (121 °C, 20 min) vor deren Einlagerung bei 2 °C bis 8 °C (Ausführungsbeispiel 4).

Die Lagerung der Vial zur Stabilitätsuntersuchung erfolgte lichtgeschützt und temperaturkontrolliert bei 5 °C ± 3 °C (Langzeitlagerung, mindestens 24 Monate), bei 15 °C ± 3 °C (Lagerung über einen mittleren Zeitraum, bis 12 Monate) und bei 25 °C ± 2 °C/40 % RH ± 5 % RH (Lagerung unter beschleunigten Bedingungen, bis 6 Monate).

## Patentansprüche

1. Flüssige pharmazeutische Lösung zur parenteralen Verabreichung, umfassend
- ein Lösungsmittel;
- Pemetrexed oder ein pharmazeutisch annehmbares Salz davon; sowie
- ein Antioxidationsmittel,
**dadurch gekennzeichnet, dass** das Antioxidationsmittel ausgewählt ist aus der Gruppe bestehend aus Acetylcystein und 2-Mercaptoethansulfonat-Natrium.

2. Lösung nach Anspruch 1, **dadurch gekennzeichnet, dass** das Lösungsmittel ausgewählt ist aus der Gruppe bestehend aus Wasser, Polyethylenglycol und Ethanol sowie aus Mischungen von zwei oder mehr der genannten Lösungsmittel.

3. Lösung nach einem der voranstehenden Ansprüche, **dadurch gekennzeichnet, dass** das Pemetrexed in der Lösung in Form seines Dinatriumsalzes gelöst enthalten ist.

4. Lösung nach einem der voranstehenden Ansprüche, **dadurch gekennzeichnet, dass** der Gehalt der Lösung an Pemetrexed bzw. an pharmazeutisch annehmbaren Salz davon 0,1 mg/ml bis 100 mg/ml beträgt, mehr bevorzugt 5 mg/ml bis 80 mg/ml, noch mehr bevorzugt 10 mg/ml bis 50 mg/ml und weiter bevorzugt 20 mg/ml bis 40 mg/ml.

5. Lösung nach einem der voranstehenden Ansprüche, **dadurch gekennzeichnet, dass** der Gehalt der Lösung an Pemetrexed bzw. an pharmazeutisch annehmbaren Salz davon 10 mg/ml, 20 mg/ml, 25 mg/ml, 40 mg/ml oder 50 mg/ml beträgt.

6. Lösung nach einem der voranstehenden Ansprüche, **dadurch gekennzeichnet, dass** das Antioxidationsmittel Acetylcystein ist.

7. Lösung nach Anspruch 6, **dadurch gekennzeichnet, dass** die Lösung einen pH-Wert in einem Bereich von 7,5 bis 11,5 aufweist, mehr bevorzugt einen pH-Wert in einem Bereich von 8,0 bis 10,5 und noch mehr bevorzugt einen pH-Wert in einem Bereich von 8,5 bis 10,5.

8. Lösung nach einem der Ansprüche 1 bis 5, **dadurch gekennzeichnet, dass** das Antioxidationsmittel 2-Mercaptoethansulfonat-Natrium ist.

9. Lösung nach Anspruch 8, **dadurch gekennzeichnet, dass** die Lösung einen pH-Wert in einem Bereich von 7,5 bis 11,5 aufweist, mehr bevorzugt einen pH-Wert in einem Bereich von 8,0 bis 10,5, noch mehr bevorzugt einen pH-Wert in einem Bereich von 8,0 bis 10,0 und weiter bevorzugt einen pH-Wert in einem Bereich von 8,3 bis 9,3.

10. Lösung nach einem der voranstehenden Ansprüche, **dadurch gekennzeichnet, dass** der Gehalt der Lösung an Antioxidationsmittel 0,1 mg/ml bis 100 mg/ml beträgt, mehr bevorzugt 0,5 mg/ml bis 20 mg/ml, noch mehr bevorzugt 1,0 mg/ml bis 5 mg/ml und weiter bevorzugt 1 mg/ml bis 3 mg/ml.

11. Lösung nach einem der voranstehenden Ansprüche, **dadurch gekennzeichnet, dass** die Lösung ferner einen oder mehrere pharmazeutische Hilfsstoffe umfasst, ausgewählt aus der Gruppe bestehend aus Salzen, Kohlehydraten zur Tonisierung, Chelatbildnern zur Komplexierung von Schwermetallen, Säuren zur pH-Wert Einstellung, Basen zur pH-Wert Einstellung, Puffersubstanzen, Konservierungsmitteln zur mikrobiellen Konservierung der Lösung.

12. Pharmazeutische Zusammensetzung zur Herstellung einer Lösung nach einem der voranstehenden Ansprüche, umfassend Pemetrexed oder ein pharmazeutisch annehmbares Salz davon sowie ein Antioxidationsmittel ausgewählt aus der Gruppe bestehend aus Acetylcystein und 2-Mercaptoethansulfonat-Natrium.

13. Zusammensetzung nach Anspruch 12, **dadurch gekennzeichnet, dass** die Zusammensetzung in fester Form vorliegt.

14. Zusammensetzung nach einem der voranstehenden Ansprüche, **dadurch gekennzeichnet, dass** die Zusammensetzung ein Lyophilisat ist.

15. Luftdicht verschlossener Behälter, enthaltend eine pharmazeutische Lösung oder eine pharmazeutische Zusammensetzung nach einem der voranstehenden Ansprüche.

## Claims

1. Liquid pharmaceutical solution for parenteral administration, comprising
- a solvent;
- pemetrexed or a pharmaceutically acceptable salt thereof; and
- an antioxidant,
**characterized in that** the antioxidant is selected from the group consisting of acetylcysteine and sodium 2-mercaptoethanesulfonate.

2. Solution according to claim 1, **characterized in that** the solvent is selected from the group consisting of water, polyethylene glycol and ethanol, and from mixtures of two or more of the solvents mentioned.

3. Solution according to either of the preceding claims, **characterized in that** the pemetrexed is present in the solution dissolved in the form of its disodium salt.

4. Solution according to any of the preceding claims, **characterized in that** the content of pemetrexed or of pharmaceutically acceptable salt thereof in the solution is 0.1 mg/ml to 100 mg/ml, more preferably 5 mg/ml to 80 mg/ml, even more preferably 10 mg/ml to 50 mg/ml and further preferably 20 mg/ml to 40 mg/ml.

5. Solution according to any of the preceding claims, **characterized in that** the content of pemetrexed or of pharmaceutically acceptable salt thereof in the solution is 10 mg/ml, 20 mg/ml, 25 mg/ml, 40 mg/ml or 50 mg/ml.

6. Solution according to any of the preceding claims, **characterized in that** the antioxidant is acetylcysteine.

7. Solution according to claim 6, **characterized in that** the solution has a pH within a range from 7.5 to 11.5, more preferably a pH within a range from 8.0 to 10.5 and even more preferably a pH within a range from 8.5 to 10.5.

8. Solution according to any of claims 1 to 5, **characterized in that** the antioxidant is sodium 2-mercaptoethanesulfonate.

9. Solution according to claim 8, **characterized in that** the solution has a pH within a range from 7.5 to 11.5, more preferably a pH within a range from 8.0 to 10.5, even more preferably a pH within a range from 8.0 to 10.0 and further preferably a pH within a range from 8.3 to 9.3.

10. Solution according to any of the preceding claims, **characterized in that** the content of antioxidant in the solution is 0.1 mg/ml to 100 mg/ml, more preferably 0.5 mg/ml to 20 mg/ml, even more preferably 1.0 mg/ml to 5 mg/ml and further preferably 1 mg/ml to 3 mg/ml.

11. Solution according to any of the preceding claims, **characterized in that** the solution further comprises one or more pharmaceutical excipients, selected from the group consisting of salts, carbohydrates for tonicity, chelating agents for complexation of heavy metals, acids for pH adjustment, bases for pH adjustment, buffer substances, preservatives for microbial preservation of the solution.

12. Pharmaceutical composition for preparation of a solution according to any of the preceding claims, comprising pemetrexed or a pharmaceutically acceptable salt thereof and an antioxidant selected from the group consisting of acetylcysteine and sodium 2-mercaptoethanesulfonate.

13. Composition according to claim 12, **characterized in that** the composition is in solid form.

14. Composition according to any of the preceding claims, **characterized in that** the composition is a lyophilizate.

15. Airtight-sealed container, containing a pharmaceutical solution or a pharmaceutical composition according to any of the preceding claims.

## Revendications

1. Solution pharmaceutique liquide destinée à une administration parentérale, comprenant
- un solvant ;
- du pémétrexed ou un sel pharmaceutiquement acceptable de celui-ci ; ainsi que
- un antioxydant,
**caractérisée en ce que** l'antioxydant est choisi dans le groupe composé de l'acétylcystéine et du 2-mercapto-éthanesulfonate de sodium.

2. Solution selon la revendication 1, **caractérisée en ce que** le solvant est choisi dans le groupe composé de l'eau, du polyéthylène glycol et de l'éthanol, ainsi que de mélanges de deux ou plusieurs desdits solvants.

3. Solution selon l'une des revendications précédentes, **caractérisée en ce que** le pémétrexed est contenu dans la solution dissous sous la forme de son sel disodique.

4. Solution selon l'une des revendications précédentes, **caractérisée en ce que** la teneur de la solution en pémétrexed ou en sel pharmaceutiquement acceptable de celui-ci est de 0,1 mg/ml à 100 mg/ml, de manière plus préférée de 5 mg/ml à 80 mg/ml, de manière encore plus préférée de 10 mg/ml à 50 mg/ml et de manière encore davantage préférée de 20 mg/ml à 40 mg/ml.

5. Solution selon l'une des revendications précédentes, **caractérisée en ce que** la teneur de la solution en pémétrexed ou en sel pharmaceutiquement acceptable de celui-ci est de 10 mg/ml, 20 mg/ml, 25 mg/ml, 40 mg/ml ou 50 mg/ml.

6. Solution selon l'une quelconque des revendications précédentes, **caractérisée en ce que** l'antioxydant est l'acétylcystéine.

7. Solution selon la revendication 6, **caractérisée en ce que** la solution présente un pH dans une plage de 7,5 à 11,5, de manière plus préférée un pH dans une plage de 8,0 à 10,5, et de manière encore plus préférée un pH dans une plage de 8,5 à 10,5.

8. Solution selon l'une des revendications 1 à 5, **caractérisée en ce que** l'antioxydant est le 2-mercapto-éthanesulfonate de sodium.

9. Solution selon la revendication 8, **caractérisée en ce que** la solution présente un pH dans une plage de 7,5 à 11,5, de manière plus préférée un pH dans une plage de 8,0 à 10,5, de manière encore plus préférée un pH dans une plage de 8,0 à 10,0, et de manière encore davantage préférée un pH dans une plage de 8,3 à 9,3.

10. Solution selon l'une des revendications précédentes, **caractérisée en ce que** la teneur de la solution en antioxydant est de 0,1 mg/ml à 100 mg/ml, de manière plus préférée de 0,5 mg/ml à 20 mg/ml, de manière encore plus préférée de 1,0 mg/ml à 5 mg/ml, et de manière encore davantage préférée de 1 mg/ml à 3 mg/ml.

11. Solution selon l'une des revendications précédentes, **caractérisée en ce que** la solution comprend en outre un ou plusieurs adjuvants pharmaceutiques choisis dans le groupe composé de sels, de glucides pour tonification, d'agents chélatants pour la formation de complexes de métaux lourds, d'acides pour l'ajustement du pH, de bases pour l'ajustement du pH, de substances tampons, de conservateurs pour la conservation microbienne de la solution.

12. Composition pharmaceutique pour la production d'une solution selon l'une des revendications précédentes, comprenant du pémétrexed ou un sel pharmaceutiquement acceptable de celui-ci, ainsi qu'un antioxydant choisi dans le groupe composé de l'acétylcystéine et du 2-mercapto-éthanesulfonate de sodium.

13. Composition selon la revendication 12, **caractérisée en ce que** la composition se présente sous forme solide.

14. Composition selon l'une des revendications précédentes, **caractérisée en ce que** la composition est un lyophilisat.

15. Conteneur fermé hermétique à l'air contenant une solution pharmaceutique ou une composition pharmaceutique selon l'une des revendications précédentes.
